(19) Europäisches Patentamt
European Patent Office
Office européen des brevets

(11) Numéro de publication: **0 092 458**
**A2**

(12) **DEMANDE DE BREVET EUROPEEN**

(21) Numéro de dépôt: **83400705.6**

(22) Date de dépôt: **07.04.83**

(51) Int. Cl.³: **C 07 D 471/04,** A 61 K 31/44
// (C07D471/04, 235/00, 221/00)

(30) Priorité: **21.04.82 FR 8206840**

(43) Date de publication de la demande: **26.10.83**
**Bulletin 83/43**

(84) Etats contractants désignés: **AT BE CH DE FR GB IT LI LU NL SE**

(71) Demandeur: **SYNTHELABO, 58, rue de la Glacière, F-75621 Paris Cedex 13 (FR)**

(72) Inventeur: **Kaplan, Jean-Pierre, 20, rue Arnoux, F-92340 Bourg-la-Reine (FR)**
Inventeur: **George, Pascal, 39, rue Henri de Vilmorin, F-94400 Vitry S/Seine (FR)**

(74) Mandataire: **Ludwig, Jacques et al, SYNTHELABO Service Brevets 58, rue de la Glacière, F-75621 Paris Cedex 13 (FR)**

(54) **Dérives d'imidazo(1,2-a)pyridine, leur préparation et leur application en thérapeutique.**

(57) Dérivés d'imidazo [1,2-α]pyridine répondant à la formule (I)

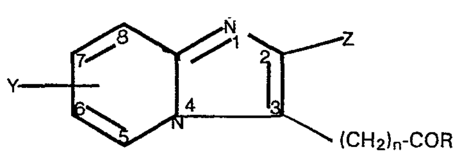

(I)

dans laquelle
n = 2, 3 ou 4
Y représente un atome d'hydrogène ou d'halogène ou un radical $C_{1-4}$alkyle,

Z représente soit un radical naphtyle, soit un radical 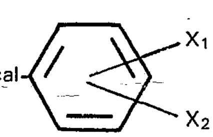

soit un radical thiényle-2, furyle-2 ou pyridyle-2,
R représente le radical OH, le radical $NR_1R_2$ dans lequel $R_1$ et $R_2$ représentent un atome d'hydrogène, un radical $C_{1-5}$alkyle, le radical allyle, le radical propargyle, le radical $C_{3-6}$cycloalkyle, le radical benzyle, le radical phényle, ou bien, $NR_1R_2$ représentent ensemble un hétérocycle.

Application en thérapeutique.

EP 0 092 458 A2

0092458

DERIVES D'IMIDAZO [1,2-*a*] PYRIDINE, LEUR PREPARATION ET LEUR APPLICATION EN THERAPEUTIQUE.

La présente invention concerne des dérivés d'imidazo [1,2-*a*] pyridine, leur préparation et leur application en thérapeutique.

Des imidazo [1,2-*a*] pyridines ont déjà été décrites dans la littérature, par exemple dans les brevets britanniques 991 589 et 1 076 089 et dans diverses publications.

Les composés de la présente invention répondent à la formule (I)

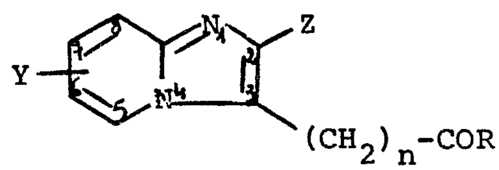 (I)

dans laquelle

n = 2, 3 ou 4

Y représente un atome d'hydrogène ou d'halogène ou un radical $C_{1-4}$alkyle,

Z représente soit un radical naphtyle, soit un radical

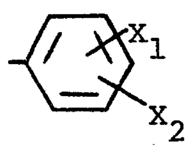 dans lequel $X_1$ et $X_2$ sont chacun indépendamment l'un de l'autre, un atome d'hydrogène ou d'halogène, un radical $C_{1-4}$ alcoxy, un radical $C_{1-6}$ alkyle, le groupe $CF_3$, $CH_3S$, $CH_3SO_2$ ou $NO_2$, soit un radical thiényle-2, furyle-2 ou pyridyle-2 pouvant porter en 5 un atome d'halogène ou le radical méthyle ou éthyle,

R représente le radical OH ou le radical $NR_1R_2$ dans lequel $R_1$ et $R_2$ représentent chacun indépendamment l'un de l'autre soit un atome d'hydrogène, soit un radical $C_{1-5}$ alkyle droit ou ramifié pouvant porter un ou plusieurs atomes d'halogène, un radical hydroxy, $N(C_{1-4}alkyl)_2$, carbamoyle ou $C_{1-4}$alcoxy, soit le radical allyle, soit le radical propargyle, soit un radical $C_{3-6}$cycloalkyle, soit le radical benzyle, soit le radical phényle,

ou bien,

$NR_1R_2$ représentent ensemble un hétérocycle comportant de 3 à 6 atomes de carbone, ou un hétérocycle de formule 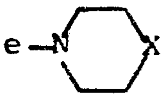

dans laquelle X est O, S, CHOR' ou NR", R' étant un atome d'hydrogène ou le radical benzyle et R" étant un atome d'hydrogène, un radical $C_{1-4}$ alkyle ou le radical phényle pouvant porter un radical méthoxy ou un atome d'halogène.

Les composés de l'invention peuvent se présenter sous forme de base libre ou de sels d'addition à des acides pharmaceutiquement acceptables.

Les composés préférés de l'invention sont ceux dans lesquels n est égal à 2 et Y est en position 6 et représente soit un atome d'halogène, soit le radical méthyle.

On peut particulierement citer parmi ces derniers les composés dans lesquels Z est soit un radical 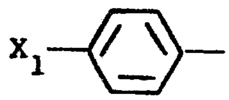 dans lequel $X_1$ est un atome d'halogène ou le radical $CH_3$, soit le radical 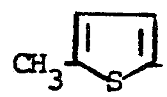, et R est soit un radical hydroxyle, soit un radical amino, soit un radical diméthylamino.

Selon l'invention, on peut préparer les composés (I) selon le schéma réactionnel suivant :

Schéma général

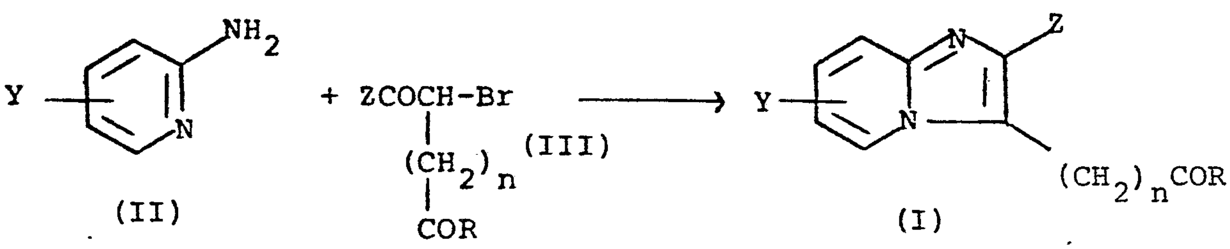

(II) (III) (I)

On fait réagir l'amino-2 pyridine (II) avec la cétone bromée (III) dans un solvant tel que l'éthanol ou le dioxanne.

Les composés dans lesquels n est 2 peuvent aussi être préparés selon le schéma réactionnel particulier suivant :

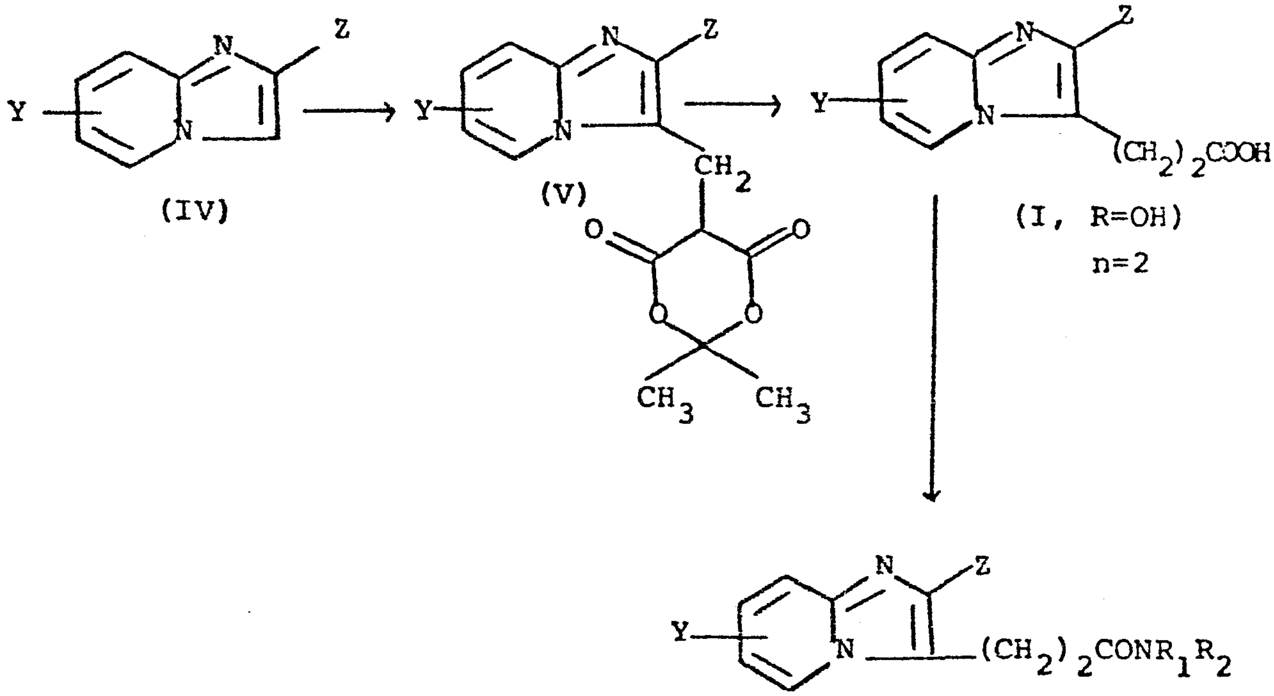

(IV) (V) (I, R=OH) n=2

(I) (I, $R=NR_1R_2$) n=2

On fait réagir le composé de départ (IV) avec la diméthyl-2,2 dioxa-1,3 cyclohexyl-dione-4,6 , en présence de formaldéhyde à 30% dans l'eau et dans un solvant tel que l'acétonitrile ; puis on chauffe le composé (V) en présence de cuivre en poudre dans de la pyridine et de l'eau pour obtenir le composé (I) dans lequel R est OH et, si on le désire, on amidifie l'acide obtenu de manière classique.

Le composé de départ (IV) est préparé par réaction entre

un composé 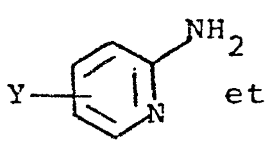 et un composé $ZCOCH_2Br$.

Les exemples suivants illustrent l'invention.

Les analyses et les spectres IR et RMN confirment la structure des composés.

**EXEMPLE 1** Acide chloro-6 (chloro-4 phényl)-2 imidazo [1,2-*a*]pyridine-3-propionique.

[Y= Cl-6, Z= 4-Cl-$C_6H_4$, R= OH]

1. On chauffe, pendant 4 heures, à 60°C, tout en agitant, 50 g (0,19 mole) de chloro-6 (chloro-4 phényl)-2 imidazo[1,2-*a*] pyridine, 21 g (0,021 mole) d'une solution à 30% de formaldéhyde dans l'eau et 27,4 g (0,19 mole) de diméthyl-2,2 dioxa-1,3 cyclohexyl-dione-2,4 dans 1 l d'acétonitrile. On filtre le mélange réactionnel froid et lave le produit avec de l'acétone puis avec du chlorure de méthylène et avec de l'éther. On sèche le produit à 45-50°C sous vide pendant 8 heures.

F = 134-135°C.

2. On chauffe pendant 3 heures à la température du reflux, sous azote, 45 g (0,107 mole) du composé obtenu sous 1, 300 $cm^3$ de pyridine et 30 $cm^3$ d'eau en présence de 0,5 g de cuivre en poudre. On filtre le milieu réactionnel à chaud, évapore à sec le filtrat . Le résidu est trituré dans 500 $cm^3$ d'une solution à 5% de chlorure d'ammonium. On filtre le solide cristallisé, et on le lave avec de l'eau. L'acide brut obtenu est mis en suspension dans 1 l d'eau auquel on ajoute 15 g de carbonate de sodium et 5 ml d'ammoniaque concentrée. On porte le mélange 30 minutes à la température du reflux tout en agitant. La suspension résultante du sel de sodium est filtrée à froid.
Le sel est ensuite introduit dans 1 l d'eau à 50-60°C, et, tout en agitant, on acidifie la solution par addition d'acide acétique jusqu'à pH= 5,5-6.
Après 1 heure d'agitation à la température ambiante, on filtre l'acide, le lave 3 fois avec de l'eau puis avec de l'acétone, et le sèche 8 heures à 100°C sous vide.

F = 278-280°C.

EXEMPLE 2 Méthyl-6 (méthyl-4 phényl)-2 imidazo[1,2-*a*]pyridine-3-propionamide.

[Y= $CH_3$-6, Z= 4-$CH_3$-$C_6H_4$, R= $NH_2$]

1. On chauffe pendant 4 heures à 55-60°C, 30 g (0,135 mole) de méthyl-6 (méthyl-4 phényl)-2 imidazo[1,2-*a*]pyridine, 19,45 g (0,135 mole) de diméthyl-2,2 dioxa-1,3 cyclohexyl-dione-2,4, 15 g (0,15 mole) de formaldéhyde à 30% dans l'eau et 500 $cm^3$ d'acétonitrile.

A froid on filtre, lave le mélange réactionnel avec de l'acétone puis le produit obtenu avec de l'éther et le sèche.

F = 182-184°C

2. On chauffe pendant 3 heures à la température du reflux, sous azote, 45 g (0,119 mole) du composé obtenu sous 1, avec 0,5 g de cuivre en poudre, dans 300 $cm^3$ de pyridine et 30 $cm^3$ d'eau. On filtre le mélange réactionnel à chaud et on ajoute 200 $cm^3$ d'une solution à 5% de chlorure d'ammonium.

On concentre le mélange réactionnel par évaporation à environ 100 $cm^3$. Après addition de 500 $cm^3$ d'eau, la suspension résultante est chauffée à 60-70°C sous agitation durant 1 heure.

On filtre à froid, et on lave le produit avec une solution de $NH_4Cl$ à 5%, puis 3 fois avec de l'eau.

Après un lavage à l'acétone, puis à l'éther, on sèche le produit 8 h à 80°C sous vide.

F = 255-257°C.

3. A une suspension de 10 g (0,034 mole) de l'acide obtenu sous 2 dans 200 $cm^3$ de T.H.F. sec, on ajoute 6,5 g (0,04 mole) de carbonyldiimidazole. Après 2 heures d'agitation à 50°C sous azote sec, on fait passer un léger courant d'ammoniac en excès. On laisse reposer le mélange réactionnel une nuit puis on l'évapore à siccité. On lave le résidu avec 200 $cm^3$ de carbonate de potassium à 5% bouillant. On filtre à chaud et renouvelle encore 2 fois ce lavage à chaud, puis on lave 3 fois avec de l'eau et sèche. On fait recristalliser le produit dans un mélange éthanol/méthyl-cellosolve 90/10, le lave avec de l'éther et le sèche à 100°C sous vide pendant 8 heures.

F = 232-233°C (déc.)

Selon le même schéma réactionnel, on a préparé à titre d'exemples les composés du tableau suivant.

TABLEAU

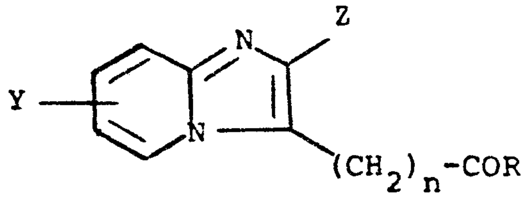

(I)

| Composé | n | Y | Z | R | F(°C) |
|---|---|---|---|---|---|
| 1 | 2 | 6-Cl | 4-Cl-$C_6H_4$ | OH | 278-280 |
| 2 | 2 | 6-Cl | 4-Cl-$C_6H_4$ | $NH_2$ | 259-260 |
| 3 | 2 | 6-Cl | 4-Cl-$C_6H_4$ | $N(CH_3)_2$ | 150,5-151 |
| 4 | 2 | 6-$CH_3$ | 4-$CH_3$-$C_6H_4$ | OH | 255-257 |
| 5 | 2 | 6-$CH_3$ | 4-$CH_3$-$C_6H_4$ | $NH_2$ | 232-233 |
| 6 | 2 | 6-$CH_3$ | 4-$CH_3$-$C_6H_4$ | $N(CH_3)_2$ | 171-172 |
| 7 | 2 | 6-$CH_3$ | 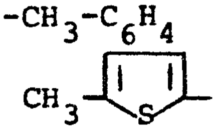 | OH | 226-228 |
| 8 | 2 | 6-$CH_3$ | 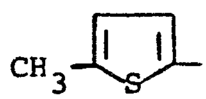 | $NH_2$ | Base:220-221<br>HCl :258-260 |
| 9 | 2 | 6-$CH_3$ | 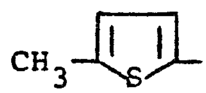 | $N(CH_3)_2$ | 141,5-142 |

Les composés de l'invention ont été soumis à des essais pharmacologiques qui ont montré leurs intéressantes propriétés pharmacologiques dans divers domaines.

La toxicité des composés a été déterminée chez la souris par voie intrapéritonéale.
La DL 50 va de 500 à 1000mg/kg.

L'activité anxiolytique a été déterminée selon le "eating test" (Stephens, R.J. (1973) Brit. J. Pharmac., 49, 146 P).
Dans ce test, les doses qui augmentent la consommation alimentaire des souris varient de 1 à 30 mg/kg, i.p.

L'activité des composés dans le domaine de la circulation cérébrale a été déterminée dans le test de l'hypoxie hypobare.
Des souris de souche CD1 sont maintenues dans une atmosphère appauvrie en oxygène, par réalisation d'un vide partiel (190 mm de mercure correspondant à 5,25% d'oxygène).
Le temps de survie des animaux est noté. Ce temps est augmenté par les agents capables de favoriser l'oxygénation tissulaire et en particulier cérébrale. Les composés étudiés sont administrés, à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100% est déterminée graphiquement. La DAM va de 0,3 à 32 mg/kg i.p.

L'activité anticonvulsivante a été déterminée selon le test de l'antagonisme vis à vis de la mortalité induite par la bicuculline chez la souris (Worms, P.,Depoortere, H. and Lloyd, K.G. (1979) Life Sci., 25, 607-614). Les produits à étudier sont injectés par voie intrapéritonéale, 30 mn avant la bicuculline (0,9 mg/kg i.v). Le critère retenu pour ce test étant la léthalité, les pourcentages de mortalité sont notés pour chaque lot, 2 heures après administration de la bicuculline (lot témoin : 100% mortalité).

Pour chaque produit la dose active 50% (DA 50 ou dose protégeant 50% d'animaux des effets léthaux de la bicuculline) est déterminée graphiquement. La DA 50 des composés de l'invention varie entre 1 à 30 mg/kg par voie i.p.

L'activité sédative ou hypnotique a été déterminée en observant l'action des composés sur l'ECoG du rat curarisé (Depoortere H., Rev. E.E.G. Neurophysiol., (1980) 10, 3, 207-214). Chez le rat curarisé, les produits à étudier ont été injectés par voie intrapéritonéale ou orale aux doses croissantes de 1 à 30 mg/kg. Ils induisent des tracés de sommeil à partir des doses allant de 1 à 10 mg/kg i.p ou p.o.

Les résultats de ces différents tests montrent que les composés de l'invention possèdent des propriétés anxiolytiques, anti-anoxiques, inductrices de sommeil, hypnotiques et anticonvulsivantes ; les composés de l'invention sont utiles pour le traitement des états d'anxiétés, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens et pour le traitement des encéphalopathies métaboliques.

Les composés de l'invention peuvent être présentés sous toute forme appropriée pour l'administration par voie orale, ou parentérale, par exemple sous forme de comprimés, de dragées, de gélules, de solutions buvables ou injectables etc.. avec tout excipient approprié.

La posologie quotidienne peut aller de 0,5 à 2000 mg.

Revendications pour les états contractants : BE, CH, DE, FR, GB, IT, LI, LU, NL, SE.

1. Dérivés d'imidazo[1,2-*a*]pyridine répondant à la formule (I)

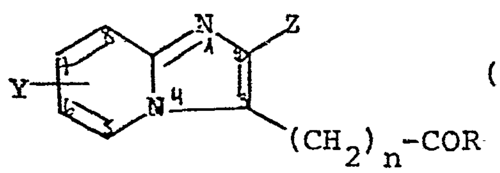

(I)

dans laquelle

n=2,3 ou 4

Y représente un atome d'hydrogène ou d'halogène ou un radical $C_{1-4}$alkyle,

Z représente soit un radical naphtyle, soit un radical 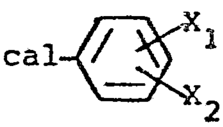 dans lequel $X_1$ et $X_2$ sont chacun indépendamment l'un de l'autre un atome d'hydrogène ou d'halogène, un radical $C_{1-4}$alcoxy, un radical $C_{1-6}$alkyle, le groupe $CF_3$, $CH_3S$, $CH_3SO_2$ ou $NO_2$, soit un radical thiényle-2, furyle-2 ou pyridyle-2 pouvant porter en 5 un atome d'halogène ou le radical méthyle ou éthyle,

R représente le radical OH ou le radical $NR_1R_2$ dans lequel $R_1$ et $R_2$ représentent chacun indépendamment l'un de l'autre soit un atome d'hydrogène, soit un radical $C_{1-5}$alkyle droit ou ramifié pouvant porter un ou plusieurs atomes d'halogène, un radical hydroxy, $N(C_{1-4}alkyl)_2$, carbamoyle ou $C_{1-4}$alcoxy, soit le radical allyle, soit le radical propargyle, soit un radical $C_{3-6}$cycloalkyle, soit le radical benzyle, soit le radical phényle,

ou bien,

$NR_1R_2$ représentent ensemble un hétérocycle comportant de 3 à 6 atomes de carbone, ou un hétérocycle de formule 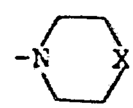

dans laquelle X est O, S, CHOR' ou NR", R' étant un atome d'hydrogène ou le radical benzyle et R" étant un atome d'hydrogène, un radical $C_{1-4}$alkyle ou le radical phényle pouvant porter un radical méthoxy ou un atome d'halogène, ainsi que leurs sels d'addition à des acides pharmaceutiquement acceptables.

2. Dérivés selon la revendication 1, dans lesquels n est 2, Y est en position 6 et représente soit un atome d'halogène, soit le radical méthyle.

3. Dérivés selon la revendication 2, dans lesquels Z est soit un radical $X_1$ 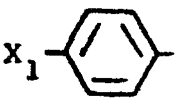 dans lequel $X_1$ est un atome d'halogène ou le radical $CH_3$, soit le radical $CH_3$ 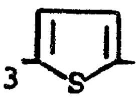 .

4. Dérivés selon la revendication 3, dans lesquels R représente un radical hydroxyle, amino ou diméthylamino.

5. Procédé de préparation des composés selon la revendication 1, procédé caractérisé en ce que l'on fait réagir une amino-2 pyridine de formule (II)

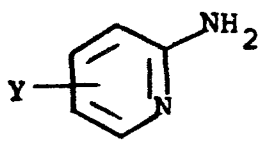

(II)

avec une cétone bromée de formule (III)

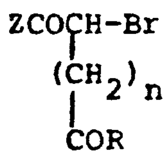

(III)

les radicaux ayant les significations données dans la revendication 1.

6. Procédé de préparation de composés selon la revendication 1, dans la formule desquels n est égal à 2, caractérisé en ce qu'on fait réagir un composé de formule IV

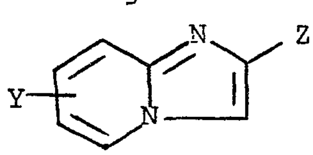

(IV)

dans laquelle Y et Z sont tels que définis à la revendication 1,

avec le diméthyl-2,2 dioxa-1,3 cyclohezyl-dione-4,6 en présence de formaldéhyde à 30 % dans l'eau et d'un solvant, puis on chauffe le composé obtenu, de formule (V)

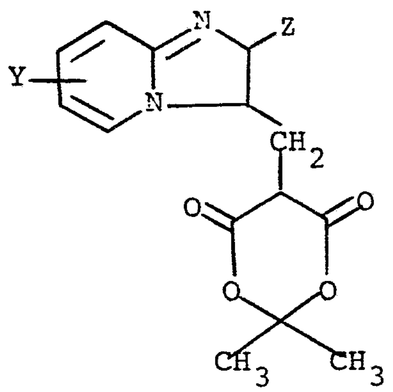

dans laquelle Y et Z sont tels que définis à la revendication 1,

en présence de cuivre en poudre dans de la pyridine et de l'eau pour obtenir le composé (I)

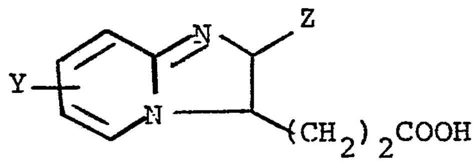

(I, R=OH
n=2)

dans laquelle Y et Z sont tels que définis à la revendication 1,

puis, si on le désire, on amidifie l'acide (I) de manière classique pour obtenir un amide de formule I dans laquelle R est un radical $NR_1R_2$, $R_1$ et $R_2$ étant tels que définis à la revendication 1.

7. Médicament caractérisé en ce qu'il contient un composé tel que spécifié dans l'une quelconque des revendications 1 à 4.

8. Composition pharmaceutique caractérisée en ce qu'elle contient un composé tel que spécifié dans l'une quelconque des revendications 1 à 4 en association avec tout excipient approprié.

Revendications pour l'état contractant : AT.

1. Procédé de préparation de dérivés d'imidazo[1,2-*a*]pyridine sous forme de bases libres ou de sels d'addition à des acides pharmaceutiquement acceptables, répondant à la formule (I)

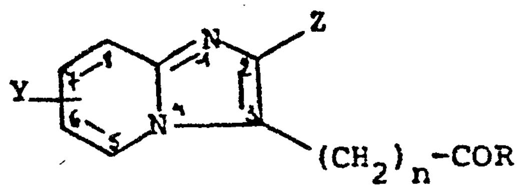 (I)

dans laquelle

n=2,3 ou 4

Y représente un atome d'hydrogène ou d'halogène ou un radical $C_{1-4}$alkyle,

Z représente soit un radical naphtyle, soit un radical 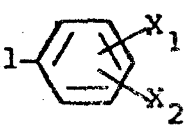

dans lequel $X_1$ et $X_2$ sont chacun indépendamment l'un de l'autre un atome d'hydrogène ou d'halogène, un radical $C_{1-4}$alcoxy, un radical $C_{1-6}$alkyle, le groupe $CF_3$, $CH_3S$, $CH_3SO_2$ ou $NO_2$, soit un radical thiényle-2, furyle-2 ou pyridyle-2 pouvant porter en 5 un atome d'halogène ou le radical méthyle ou éthyle,

R représente le radical OH ou le radical $NR_1R_2$ dans lequel $R_1$ et $R_2$ représentent chacun indépendamment l'un de l'autre soit un atome d'hydrogène, soit un radical $C_{1-5}$alkyle droit ou ramifié pouvant porter un ou plusieurs atomes d'halogène, un radical hydroxy, $N(C_{1-4}alkyl)_2$, carbamoyle ou $C_{1-4}$alcoxy, soit le radical allyle, soit le radical propargyle, soit un radical $C_{3-6}$cycloalkyle, soit le radical benzyle, soit le radical phényle,

ou bien,

$NR_1R_2$ représentent ensemble un hétérocycle comportant de 3 à 6 atomes de carbone, ou un hétérocycle de formule 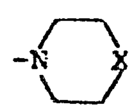

dans laquelle X est O, S, CHOR' ou NR", R' étant un atome d'hydrogène ou le radical benzyle et R" étant un atome d'hydrogène, un radical $C_{1-4}$alkyle ou le radical phényle pouvant porter un radical méthoxy ou un atome d'halogène, procédé caractérisé en ce qu'on fait réagir une amino-2 pyridine de formule (II)

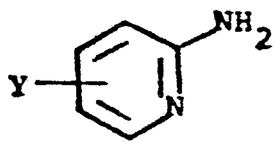

(II)

avec une cétone bromée de formule (III)

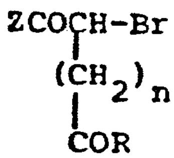

(III)

les radicaux ayant les significations données ci-dessus.

2. Procédé de préparation de dérivés d'imidazo[1,2-*a*]pyridine sous forme de bases libres ou de sels d'addition à des acides pharmaceutiquement acceptables, répondant à la formule (I)

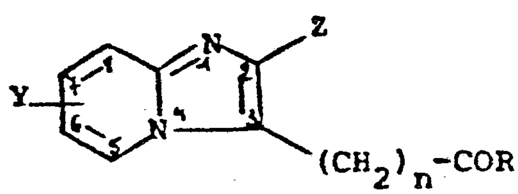

(I)

dans laquelle
n=2,
Y représente un atome d'hydrogène ou d'halogène ou un radical $C_{1-4}$alkyle,
Z représente soit un radical naphtyle, soit un radical 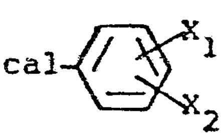

dans lequel $X_1$ et $X_2$ sont chacun indépendamment l'un de l'autre un atome d'hydrogène ou d'halogène, un radical $C_{1-4}$alcoxy, un radical $C_{1-6}$alkyle, le groupe $CF_3$, $CH_3S$, $CH_3SO_2$ ou $NO_2$, soit

un radical thiényle-2, furyle-2 ou pyridyle-2 pouvant porter en 5 un atome d'halogène ou le radical méthyle ou éthyle,

R représente le radical OH ou le radical $NR_1R_2$ dans lequel $R_1$ et $R_2$ représentent chacun indépendamment l'un de l'autre soit un atome d'hydrogène, soit un radical $C_{1-5}$alkyle droit ou ramifié pouvant porter un ou plusieurs atomes d'halogène, un radical hydroxy, $N(C_{1-4}alkyl)_2$, carbamoyle ou $C_{1-4}$alcoxy, soit le radical allyle, soit le radical propargyle, soit un radical $C_{3-6}$cycloalkyle, soit le radical benzyle, soit le radical phényle,

ou bien,

$NR_1R_2$ représentent ensemble un hétérocycle comportant de 3 à 6 atomes de carbone, ou un hétérocycle de formule 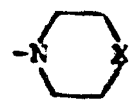

dans laquelle X est O, S, CHOR' ou NR", R' étant un atome d'hydrogène ou le radical benzyle et R" étant un atome d'hydrogène, un radical $C_{1-4}$alkyle ou le radical phényle pouvant porter un radical méthoxy ou un atome d'halogène, procédé caractérisé en ce qu'on fait réagir un composé de formule IV

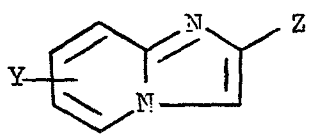

(IV)

dans laquelle Y et Z sont tels que définis ci-dessus, avec la diméthyl-2,2 dioxa-1,3 cyclohexyl-dione-4,6 en présence de formaldéhyde à 30 % dans l'eau et d'un solvant, puis on chauffe le composé obtenu, de formule (V)

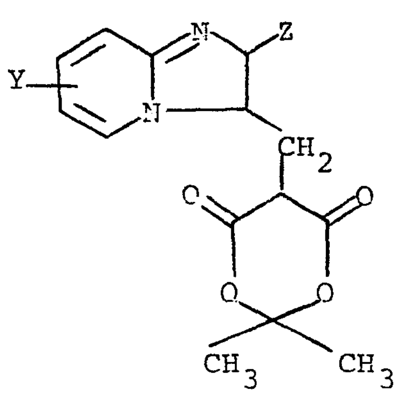

dans laquelle Y et Z sont tels que définis ci-dessus, en présence de cuivre en poudre dans de la pyridine et de l'eau pour obtenir le composé (I)

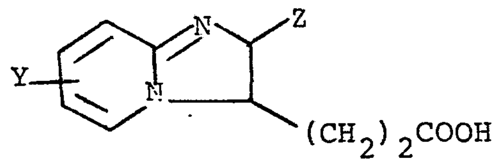

(I, R=OH n=2)

dans laquelle Y et Z sont tels que définis ci-dessus, puis, si on le désire, on amidifie l'acide (I) de manière classique pour obtenir un amide de formule I dans laquelle R est un radical $NR_1R_2$, $R_1$ et $R_2$ étant tels que définis ci-dessus.

3. Procédé selon l'une des revendications 1 et 2 caractérisé en ce que, dans les formules (I), (II), (III), (IV) et (V), n est égal à 2, Y est en position 6 et représente soit un atome d'halogène, soit le radical méthyle.

4. Procédé selon la revendication 3, caractérisé en ce que, dans les formules (I) et (II), Z est soit un radical 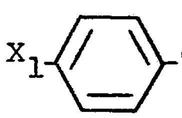 dans lequel $X_1$ est un atome d'halogène ou le radical $CH_3$, soit le radical 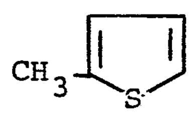

5. Procédé selon la revendication 4, caractérisé en ce que, dans la formule (I), R représente un radical hydroxyle, amino ou diméthylamino.